# EUROPEAN PATENT APPLICATION

(11) **EP 4 768 565 A1**
(43) Date of publication of application: **01.07.2026**
(21) Application number: 25217908.0
(22) Date of filing: 24.11.2025
(51) Int. Cl.: C12M 1/107, C12M 1/33, C12M 1/00, C12P 5/02

(54) **2-PHASE ANAEROBIC DIGESTION SYSTEM WITH INCREASED BIOGAS PRODUCTION**

(30) Priority: 27.12.2024 KR 20240199298
(71) Applicant: Choilab Inc., Seoul 01811 (KR)
(72) Inventor: CHOI, Yong Keun, 01776 Seoul (KR); PARK, Sae Rom, 16434 Suwon-si, Gyeonggi-do (KR)
(74) Representative: Jung, Minkyu

(57) **Abstract**

The present invention relates to a 2-phase anaerobic digestion system with increased biogas production capable of increasing the efficiency of anaerobic digestion of organic waste and the amount of biogas production by applying biochar to a 2-phase anaerobic digestion system in which acidogenesis and methanogenesis are carried out separately.

## Description

### [Technical Field]

The present invention relates to a 2-phase anaerobic digestion system with increased biogas production, and more specifically, to an anaerobic digestion system and an anaerobic digestion method capable of increasing the efficiency of anaerobic digestion of organic waste and the amount of biogas production by applying biochar to a 2-phase anaerobic digestion system in which acidogenesis and methanogenesis are carried out separately.

The present disclosure was made with the support of the Ministry of Climate, Energy, Environment(MCEE) of the Republic of Korea, under Project No. 202303-0401, which was conducted under the research subject named "Demand-based carbon neutral water technology demonstration support project", within the research project entitled "Demonstration of biogas production technology using biochar derived from sewage sludge" by the Choi Lab Co., Ltd. under the management of the National Water Industry Cluster Project Office, from May 31, 2023 to December 31, 2025.

This application claims priority to and the benefit of Korean Patent Application No. 10-2024-0199298 filed in the Korean Intellectual Property Office on 27 Dec. 2024, the disclosure of which is incorporated herein by reference.

### [Background Art]

Biogas is produced through an anaerobic digestion process using microorganisms that treat organic waste such as livestock manure, food waste, and sewage sludge. Such biogas mainly consists of methane and carbon dioxide, with small amounts of hydrogen sulfide and siloxane as impurities. Biogas production can treat over 65 million tons of organic waste generated annually without resorting to landfill or incineration of the organic waste and produce methane gas usable as an energy source, thus drawing global attention. In particular, in Korea, due to the enforcement of the "Act on Promotion of Production and Utilization of Biogas from Organic Waste" (commonly referred to as the "Biogas Act") in December 2023, a penalty is imposed if biogas production goals are not accomplished. Accordingly, technologies for increasing the amount of biogas production to ensure sufficient biogas output are needed.

### [Patent Literature]

### [Patent Documents]

Patent Document 1: Korean Patent No. 10-0948287

### [Disclosure]

### [Technical Problem]

Accordingly, the inventors of the present invention have found that, in an anaerobic digestion process, the digestion efficiency of organic waste is improved when acidogenesis and methanogenesis are separate such that fermentation products generated from each process are treated individually. In addition, the input of biochar in the process can further increase biogas production.

Accordingly, an objective of the present invention is to provide a 2-phase anaerobic digestion system capable of producing biogas through anaerobic digestion of organic waste.

Another objective of the present invention is to provide a method for producing biogas through 2-phase anaerobic digestion of organic waste.

### [Technical Solution]

The present invention relates to a 2-phase anaerobic digestion system with increased biogas production, which can carry out acidogenesis and methanogenesis of organic waste sequentially, thereby improving the biogas production efficiency.

Hereinafter, the present invention will be described in further detail.

According to one aspect of the present invention, there is provided a 2-phase anaerobic digestion system including: an acidogenic reactor in which acidogenesis of organic waste is performed; and a methanogenic reactor in which methanogenesis of an acidogenic liquid generated from the acidogenic reactor is performed.

In the present invention, the term "organic waste" refers to waste mainly composed of organic matter derived from nature or from human industrial activities, and may include one or more selected from the group including sludge, livestock manure, food waste, agricultural by-products, forestry by-products, fishery by-products, and microalgae. Examples of agricultural by-products include coffee grounds, oil cakes, straw, and rice husks. Examples of forestry by-products include fallen leaves, bark, fruits, weeds, and grass, and examples of fishery by-products include shells, crustacean shells, viscera, fish scales, and fish heads, but are not limited thereto.

In one embodiment of the present invention, the acidogenesis unit or the methanogenesis unit may be configured such that biochar is introduced during the acidogenesis or methanogenesis process.

In the present invention, "biochar" refers to an organic carbon mass obtained by pyrolyzing organic waste in a reductive atmosphere of low oxygen or no oxygen. For example, the biochar may be produced by pyrolyzing one or more organic wastes selected from the group including sludge, livestock manure, food waste, agricultural by-products, forestry by-products, fishery by-products, and microalgae.

Introduction of an appropriate amount of biochar into the anaerobic digestion process may provide minerals, adsorb inhibitory factors within the digester, and immobilize microorganisms. As a result, the activity of fermentation microorganisms may be enhanced, the lag phase at the beginning of the anaerobic digestion process may be shortened, and the degradation efficiency of the feeds and methane yield may be increased.

When an excessive amount of biochar is introduced, electron transfer between microorganisms may be inhibited, and essential components required for anaerobic digestion may be extensively adsorbed onto the biochar, thereby suppressing the anaerobic digestion process, which may result in reduced feed decomposition efficiency and methane yield.

Accordingly, in the anaerobic digestion of organic waste using the 2-phase anaerobic digestion system according to the present invention, the system efficiency can be improved through the introduction of an appropriate amount of biochar. For example, the biochar may be introduced at a concentration ranging from 0.5 to 20 g/L, such as 0.5 g/L, 1 g/L, 2 g/L, 5 g/L, 10 g/L, or 20 g/L.

In one embodiment of the present invention, the system may further include a receiving unit in which organic waste introduced as a feeds for anaerobic digestion is introduced.

In one embodiment of the present invention, the system may further include a pretreatment unit in which the organic waste is pretreated.

In one embodiment of the present invention, the pretreatment unit performs one or more operations selected from the group including drying, crushing, composting, microbial decomposition, pyrolysis, screening, and sorting of the organic waste.

In one embodiment of the present invention, the system may further include a carbonization unit in which biochar is produced by carbonizing the organic waste.

In one embodiment of the present invention, the carbonization unit operates at a temperature ranging from 300°C to 800°C.

In one embodiment of the present invention, the system may further include a collection unit for collecting biogas generated from the acidogenic reactor or the methanogenic reactor.

In the present invention, the term "biogas" refers to gas generated when organic matter is decomposed by microorganisms under anaerobic conditions such as anoxic or hypoxic environments. Biogas mainly includes methane and carbon dioxide but may further include gases such as nitrogen, hydrogen, oxygen, hydrogen sulfide, moisture, and ammonia. In this case, gases other than methane, which can be used as energy sources or raw materials for chemical synthesis, are treated as impurities in the biogas production process and may be separated from methane during purification and upgrading processes after biogas collection. However, in consideration of the fact that gases such as carbon dioxide, nitrogen, hydrogen, and oxygen are also widely used in various industrial fields, purification and upgrading processes may be carried out for each gas so that they can be converted into high-purity gas resources.

In one embodiment of the present invention, the collection unit performs compression storage, purification, or upgrading of the biogas.

In one embodiment of the present invention, the system further includes a by-product treatment unit for treating anaerobic digestion by-products generated from the acidogenic reactor or the methanogenic reactor.

In one embodiment of the present invention, the by-product treatment unit performs one or more operations selected from drying, composting, aging, or crushing of the anaerobic digestion by-products.

Another aspect of the present invention relates to a 2-phase anaerobic digestion method includes: an acidogenesis step of subjecting organic waste to acidogenesis; and a methanogenesis step of subjecting an acidogenic solution obtained from the acidogenesis step to methanogenesis.

In one embodiment of the present invention, in the acidogenesis step or the methanogenesis step, biochar may be further added to the organic waste or the acidogenic liquid in the acidogenesis step or the methanogenesis step.

### [Advantageous Effects]

The present invention relates to a 2-phase anaerobic digestion system with increased biogas production, which can separately perform acidogenesis and methanogenesis processes of organic waste, thereby improving both the anaerobic digestion efficiency and the biogas production efficiency.

### [Description of Drawings]

FIG. 1 is a schematic diagram illustrating the configuration of a 2-phase anaerobic digestion system according to an embodiment of the present invention.
FIG. 2 is a schematic flowchart illustrating a 2-phase anaerobic digestion method according to an embodiment of the present invention.
FIG. 3 is a graph comparing the total mineral content of six types of biochar prepared from different raw materials according to an embodiment of the present invention.
FIG. 4 is a graph comparing the relative biogas production yield when the six types of biochar prepared from different raw materials are introduced into either a methanogenesis reactor (AD) or an acidogenesis reactor (Acid), according to an embodiment of the present invention.
FIG. 5 is a graph comparing the relative methane production yield when the six types of biochar prepared from different raw materials are introduced into either the methanogenesis reactor (AD) or the acidogenesis reactor (Acid), according to an embodiment of the present invention.
FIG. 6 is a graph comparing the relative biogas production yield over time when biochar prepared from sludge is introduced into either the methanogenesis reactor (AD) or the acidogenesis reactor (Acid), according to an embodiment of the present invention.

### [Mode for Invention]

Hereinafter, embodiments of the present invention will be described in detail with reference to the accompanying drawings. However, the detailed descriptions provided below along with the drawings are merely illustrative of exemplary embodiments of the present invention and do not represent the only forms in which the present invention can be realized.

In some cases, well-known structures may be omitted to avoid obscuring the concept of the present invention. Throughout this specification, the same reference numerals refer to the same components.

Throughout this specification, unless otherwise specified, "%" used to indicate concentrations of specific substances shall mean percent weight/weight (% w/w) for solid/solid, percent weight/volume (% w/v) for solid/liquid, and percent volume/volume (% v/v) for liquid/liquid.

Unless otherwise specified, all numbers, values, and/or expressions used to describe ingredients, reaction conditions, and contents in this specification should be understood as being modified by the term "about" as the numerical values reflect inherent uncertainties in measurement.

In addition, when a numerical range is disclosed in this specification, the range is continuous and includes all values between and including the minimum and maximum values unless otherwise indicated.

Further, the term "or" as used herein is intended to be inclusive rather than exclusive, unless explicitly stated otherwise. For example, "X comprises A or B" shall mean: X comprises A, or comprises B, or comprises both A and B, unless the context clearly dictates otherwise.

Moreover, throughout this specification, when a part is described as "including" a component, it does not exclude the possibility of including other components, unless otherwise stated.

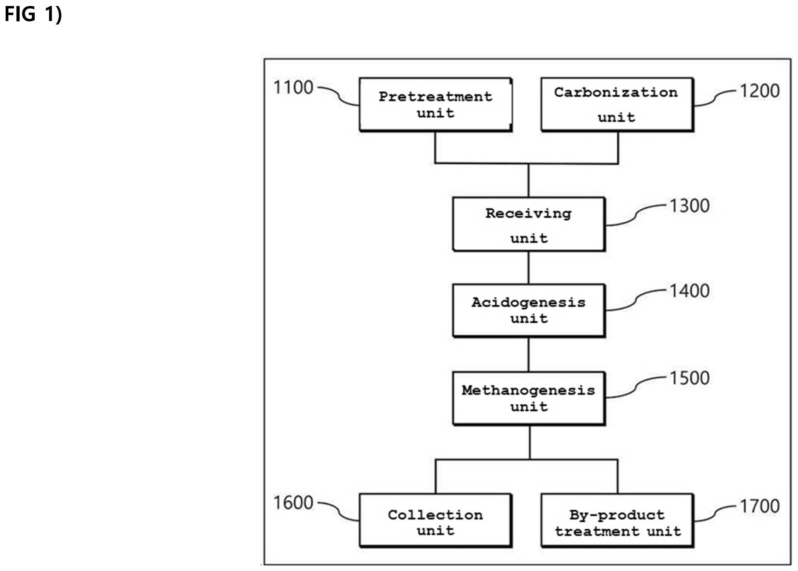

FIG. 1 is a schematic diagram illustrating the configuration of a 2-phase anaerobic digestion system according to an embodiment of the present invention.

Referring to FIG. 1, the 2-phase anaerobic digestion system 1000 according to the present invention may include an acidogenesis unit 1400 and a methanogenesis unit 1500. In the acidogenesis unit 1400 and the methanogenesis unit 1500, fermentation of organic waste by obligate anaerobes or facultative anaerobes may be carried out, thereby producing biogas including methane and carbon dioxide.

To carry out anaerobic digestion in the 2-phase anaerobic digestion system 1000, raw materials or feeds must be introduced into the acidogenesis unit 1400. In the system according to an embodiment of the present invention, organic waste may be used as the feeds for anaerobic digestion. The introduction of the feeds for anaerobic digestion may be performed in a batch type or a continuous type. To improve the digestion efficiency of the feeds for anaerobic digestion, feeds may be agitated by an agitator (not shown) arranged at the bottom, side, or top of the acidogenesis unit 1400 or the methanogenesis unit 1500.

In the acidogenesis unit 1400, not only acidogenesis but also hydrolysis and acetogenesis reactions may be carried out. For this purpose, the microbial community in the acidogenesis unit 1400 may include hydrolytic microorganisms, acidogenic microorganisms, and acetogenic microorganisms wholly or partially. The hydrolytic microorganisms may be one or more genera selected from the group including Cellulomonas, Clostridium, Bacillus, Thermomonospora, Ruminococcus, Bacteroides, Streptomyces, and Microbispora, the acidogenic microorganisms may be one or more genera selected from the group including Lactobacillus, Escherichia, Staphylococcus, Streptococcus, Pseudomonas, Selenomonas, Sarcina, Desulfobacter, Desulforomonas, and Desulfovibrio, and the acetogenic microorganisms may be Acetobacterium or Sporomusa, but are not limited thereto.

Due to the action of various microbial communities in the acidogenesis unit 1400, the organic waste introduced into the acidogenesis unit 1400 may undergo physical and chemical transformation and be decomposed into: gases such as carbon dioxide, hydrogen, nitrogen, ammonia, and hydrogen sulfide; liquids such as fatty acids, volatile fatty acids, aldehydes, alcohols, and acetic acid; or solids such as silicon dioxide, silicates, and siloxanes. The gas, liquid, or solid components generated inside the acidogenesis unit 1400 may either be introduced into the methanogenesis unit 1500 as methanogenic reaction liquid or be discharged from the system 1000 as by-products of the acidogenesis process.

In the methanogenesis unit 1500, a methanogenesis reaction may be carried out. To ensure that the methanogenesis reaction is performed intensively, the microbial community inside the methanogenesis unit 1500 may include methanogenic archaea. The methanogenic archaea may be one or more genera selected from the group including Methanobacterium, Methanobrevibacter, Methanococcus, Methanogenium, Methanospirillum, Methanomicrobium, Methanosarcina, Methanomassiliicoccus, Methanogranum, and Methanoculleus, but are not limited thereto.

When the acidogenic solution generated in the acidogenesis unit 1400 is introduced into the methanogenesis unit 1500 as a methanogenic reaction liquid, acetic acid, carbon dioxide, hydrogen, or ethanol contained in the reaction liquid may be metabolized by methanogenic archaea and converted into methane. Gaseous components such as nitrogen, ammonia, and hydrogen sulfide, liquid components such as fatty acids and aldehydes, and solid components such as silicon dioxide, silicates, and siloxanes that are not metabolized by methanogenic archaea may be discharged from the system 1000 as by-products of the methanogenesis process.

The acidogenic solution generated in the acidogenesis unit 1400 may be supplied continuously or intermittently to the methanogenesis unit 1500. The supply amount of the acidogenesis liquor may be adjusted such that the acidogenesis liquor supplied to the methanogenesis unit and the methanogenic reaction liquor remaining therein maintain a predetermined volume ratio. For example, the volume ratio may be controlled to 10:1, 5:1, 1:1, 1:3, 1:5, 1:10, 1:15, 1:20, 1:25, 1:30, or 1:50, and more specifically, it may be 1:30, but is not limited thereto.

The operating conditions of the acidogenesis unit 1400 and the methanogenesis unit 1500 may be adjusted based on the characteristics of the microbial communities in each unit. For example, the acidogenesis unit 1400 and the methanogenesis unit 1500 may operate at a temperature ranging from 20 to 50°C. The acidogenesis unit 1400 may operate under aerobic conditions with an appropriate amount of oxygen, while the methanogenesis unit 1500 may operate under hypoxic to anoxic conditions, and ultimately under anaerobic conditions. To improve the decomposition and digestion efficiency of organic waste in the system 1000, biochar may be introduced into the acidogenesis unit 1400 or the methanogenesis unit 1500. The introduction of biochar may promote the decomposition and digestion of the feeds for anaerobic digestion by supplying minerals, adsorbing acidogenesis or methanogenesis inhibitors, and immobilizing fermentation microorganisms through the biochar, thereby increasing the biogas production yield of the 2-phase anaerobic digestion system 1000. The biochar may be introduced at a concentration of 0.5 to 20 g per 1 L of the acidogenic or methanogenic reaction liquid. Considering that the anaerobic feeds consisting of organic waste are first introduced into the acidogenesis unit 1400, undergo acidogenesis, and are subsequently transferred to the methanogenesis unit 1500, the biochar may be introduced into the acidogenesis unit 1400 or into both the acidogenesis unit 1400 and the methanogenesis unit 1500 to promote biodegradation and acidogenesis of the feeds.

The system 1000 of the present invention may further include a receiving unit 1300. Organic waste introduced as feeds for anaerobic digestion may be accommodated in the receiving unit 1300, and a predetermined amount of feeds may be supplied from the receiving unit 1300 to the acidogenesis unit 1400 according to the operation environment of the acidogenesis unit 1400. Therefore, when the system 1000 further includes the receiving unit 1300, the amount of organic waste introduced into the system 1000 may be controlled based on the microbial activity, microbial composition, environmental conditions for microbial proliferation, and the residual amount of the anaerobic feeds in the acidogenesis unit 1400.

The system 1000 of the present invention may further include a pretreatment unit 1100. In the pretreatment unit 1100, pretreatment of the organic waste may be performed. For example, primary treatment such as drying, pulverizing, screening, and classification of the organic waste may be conducted, and then, the dried, pulverized, and classified organic waste may be secondarily subjected to a composting or decomposition process using aerobic microorganisms at a temperature of 20 to 50°C, or alternatively, to a pyrolysis process at a temperature of 100°C or higher, but is not limited thereto.

The system 1000 of the present invention may further include a carbonization unit 1200. In the carbonization unit 1200, the organic waste may be converted into biochar through pyrolysis and gasification. As the carbonization materials in the carbonization unit 1200, one or more organic wastes selected from the group including sludge, livestock manure, food waste, agricultural by-products, forestry by-products, marine by-products, and microalgae may be introduced. The organic waste that has undergone drying, pulverization, and screening in the pretreatment unit 1100 may be introduced into the carbonization unit 1200.

To obtain biochar from the organic waste introduced into the carbonization unit 1200, the waste may be heated at a temperature of 300 to 800°C under low-oxygen or anoxic conditions for one to five hours. By controlling the carbonization temperature and time, the physicochemical properties of the biochar can be adjusted. For example, when the organic waste is carbonized at 550°C for about one hour, the pH of the biochar may be about 8 to 9. When carbonized at 550°C for about two hours, the pH may be about 10 to 11. Therefore, biochar suitable for the reaction environment of the acidogenesis unit 1400 or the methanogenesis unit 1500 may be obtained by adjusting the carbonization conditions in the carbonization unit 1200.

In the meantime, the biochar obtained from the carbonization unit 1200 may be further formulated. For example, the biochar may be formulated into powder, granules, or pellets. More specifically, the biochar may be formed into cylindrical pellets having a diameter of 0.3 to 3 cm and a height of 0.3 to 3 cm. During the formulation process of the biochar, biodegradable biopolymers such as alginate, carrageenan, chitosan, carboxymethyl cellulose (CMC), hydroxypropyl cellulose (HPC), and hydroxypropyl methylcellulose (HPMC) may be added to increase viscosity and binding force between biochar particles. Depending on the charge characteristics of the biopolymers, cationic, anionic, or neutral curing aids may also be added.

The system 1000 of the present invention may further include a collection unit 1600. In the collection unit 1600, biogas generated from the acidogenesis unit 1400 or the methanogenesis unit 1500 may be collected. The collected biogas may be compressed and stored under high pressure for ease of gas transport and storage. Prior to or following compression, the biogas may be subjected to filtration and adsorption processes to undergo purification and upgrading of the biogas.

The system 1000 of the present invention may further include a by-product treatment unit 1700. In the by-product treatment unit 1700, by-products of anaerobic digestion generated from the acidogenesis unit 1400 or the methanogenesis unit 1500 may be treated. When treating the anaerobic digestion by-products, solid residues or liquid filtrates may be mixed to form sludge-like by-products. The sludge-like by-products may be dried, composted, or matured and then crushed to a predetermined particle size to be converted into solid organic waste. Therefore, the anaerobic digestion by-products generated in the system 1000 may be recycled as anaerobic digestion feeds or as carbonization feeds for biochar production in the carbonization unit 1200.

The system 1000 of the present invention may further include a control unit (not shown) for controlling the operation of the system.

The control unit may be wired or wirelessly connected to each unit of the system 1000 and may control individual operations thereof. For example, the control unit may analyze the characteristics of the organic waste supplied to the system 1000, and then control the pretreatment unit 1100 to perform a pretreatment process appropriate for the organic waste. Based on the analysis results of the supplied organic waste or the pretreated waste, and internal operating conditions of the acidogenesis unit 1400 or methanogenesis unit 1500, the control unit may control the carbonization unit 1200 to produce biochar under appropriate conditions. Additionally, after analyzing the characteristics of the anaerobic digestion feeds supplied to the system 1000 or pretreated by the pretreatment unit 1100, the control unit may collect information with the operating environments of the acidogenesis unit 1400 to control the receiving unit 1300 so that an appropriate amount of feeds may be introduced into the acidogenesis unit 1400. Furthermore, after analyzing the characteristics of the acidogenic solution produced in the acidogenesis unit 1400 and collecting the analysis results and the information on internal operating environments of the methanogenesis unit 1500, the control unit may control the acidogenesis unit 1400 to ensure that the methanogenic reaction liquid is appropriately transferred to the methanogenesis unit 1500 from the acidogenesis unit 1400. The control unit may also analyze the internal operating conditions of the acidogenesis unit 1400 or methanogenesis unit 1500 and, based on the analysis results, calculate the appropriate amount of biogas to be collected and the amount of anaerobic digestion by-product to be processed at specific time points, thereby controlling the operation of the collection unit 1600 and the by-product treatment unit 1700.

The control of the system 1000 via the control unit may be based on operational data generated and collected during system operation, as well as informational data pre-input before operation. The control unit may include a separate learning module (not shown) that comprehensively performs machine learning on operational data and informational data including the pretreatment characteristics of organic waste, production characteristics of biochar, anaerobic digestion characteristics of the feeds, and the properties of biogas and by-products generated during anaerobic digestion, thereby deriving optimal operating conditions for the system 1000 and controlling the operation of each unit of the system 1000. The operational data and informational data may include internal operating environment information such as internal temperature, humidity, pressure, feed weight, feed distribution, and feed flowability in the acidogenesis unit 1400, internal operating environment information on internal temperature, humidity, pressure, reaction liquid weight, and reaction liquid flowability in the methanogenesis unit 1500, information on composition, impurity content, dryness, size, and weight of the organic waste, information on raw material, degree of carbonization, pH, formulation type, size, and weight of the biochar, information on composition, density, temperature, and pressure of the biogas, and information on the composition, form, dryness, size, and weight of the by-products. However, the present invention is not limited thereto.

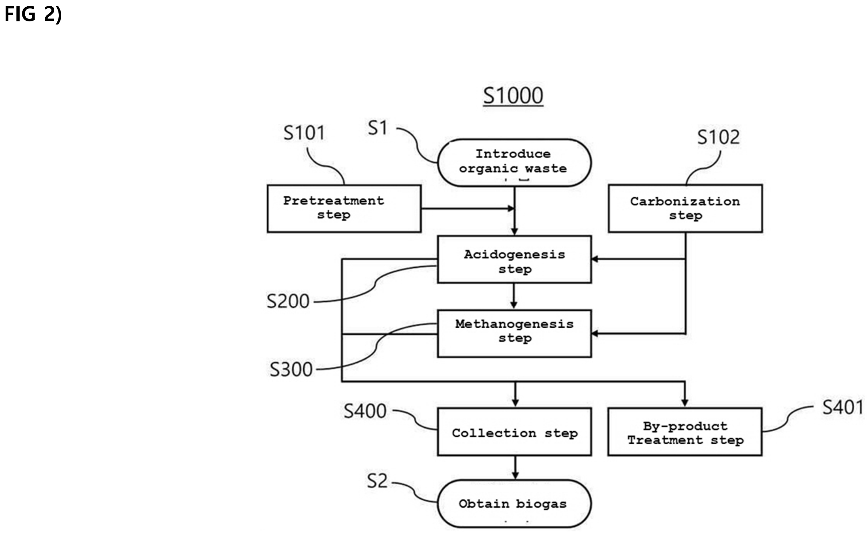

FIG. 2 is a schematic flowchart illustrating a 2-phase anaerobic digestion method according to an embodiment of the present invention.

Referring to FIG. 2, the biogas production method (S1000) according to the present invention may include: a feed input step (S1) of introducing feeds including organic waste, an acidogenesis step (S200) for fermenting the feeds, a methanogenesis step (S300) for fermenting the acidogenic solution, and a gas collection step (S400) of collecting gases generated during the acidogenesis or methanogenesis processes. Through the 2-phase anaerobic digestion method, biogas may be obtained (S2).

The acidogenesis step (S200) or the methanogenesis step (S300) may be carried out under hypoxic to anaerobic conditions at 20 to 50°C, and the reaction temperature and oxygen conditions may be adjusted according to the characteristics of the microorganisms used for acidogenesis or methanogenesis.

The anaerobic digestion in the acidogenesis step (S200) or the methanogenesis step (S300) may be performed in a batch-type, a continuous-type, or a semi-continuous type. To improve fermentation efficiency in each step, the feeds or the reaction liquid may be agitated in part or in whole during the acidogenesis step (S200) or the methanogenesis step (S300).

To enhance the decomposition and digestion efficiency of organic waste, biochar may be additionally introduced in the acidogenesis step (S200) or the methanogenesis step (S300). If prolonged contact time between the reactants/microorganisms and the biochar is required, biochar may be added during the acidogenesis step (S200). Conversely, if prolonged contact time is not necessary, biochar may be added during the methanogenesis step (S300). When the longest possible contact time is desired, biochar may be added in both the acidogenesis and methanogenesis steps. The amount of biochar may be appropriately adjusted within a range of 0.5 to 20 g/L.

The gas collected during the collection step (S400) may contain not only methane and carbon dioxide but also impurities such as water vapor, hydrogen sulfide, nitrogen, and ammonia. Therefore, a purification process may be additionally carried out to increase the purity of the biogas. The purification process may be based on gas filtration and adsorption principles to achieve upgrading of the biogas including methane and carbon dioxide. The purified or unpurified biogas may be compressed and stored under high pressure for ease of transport and storage.

The biogas production method (S1000) of the present invention may further include a pretreatment step (S101) in which pretreatment of the organic waste, used as the feeds for anaerobic digestion, is carried out before the acidogenesis step (S200).

In the pretreatment step (S101), operations such as drying, pulverizing, screening, or classifying the waste may be carried out to improve the suitability of the waste for input. Additionally, composting or decomposition using aerobic microorganisms or thermal pyrolysis at high temperatures may be performed to improve digestion efficiency, but the process is not limited thereto.

The biogas production method (S1000) of the present invention may further include a carbonization step (S102) for obtaining biochar to be introduced into the anaerobic digestion process from organic waste. In the carbonization step (S102), one or more types of organic waste selected from the group including sludge, livestock manure, food waste, agricultural by-products, forestry by-products, fishery by-products, and microalgae may be pyrolyzed by heating for one to five hours at 300 to 800°C under hypoxic to anaerobic conditions to be converted into biochar.

The biogas production method (S1000) of the present invention may further include a by-product treatment step (S401) of treating anaerobic digestion by-products generated together with gases in the acidogenesis step (S200) or methanogenesis step (S300). In the by-product treatment step (S401), a sludge-like mixture of liquid and solid by-products generated during anaerobic digestion may be dried, composted, matured, or pulverized and thereby converted into organic waste.

Hereinafter, the present invention will be described in more detail with reference to the following examples. However, the following examples are provided merely to illustrate the present invention, and are not intended to limit the scope of the invention.

### Example 1. Preparation of Biochar

Biochar was prepared for use in the anaerobic digestion process of organic waste.

Specifically, leaves, microalgae, spent coffee grounds, rice husks, lawn grass, and sewage sludge were collected from sewage treatment plants, municipal waste treatment facilities, domestic waste treatment plants, and farms. Each raw material was dried and then pulverized to a particle size of 10 mm or less. The pulverized raw materials were fed into a pyrolysis reactor, and pyrolysis was carried out at 550°C for two hours under a nitrogen atmosphere. The resulting product was washed with distilled water to remove impurities and then subjected to post-drying at 105°C for 24 hours to yield a total of six types of biochar.

The resulting six types of biochar were designated as follows:
Leaf-derived biochar: M-BC
Microalgae-derived biochar: SP-BC
Spent coffee grounds-derived biochar: CF-BC
Rice husk-derived biochar: RH-BC
Lawn grass-derived biochar: KB-BC
Sludge-derived biochar: Slu-BC

### Example 2. Measurement of Mineral Content in Biochar

The total mineral content in the six types of biochar prepared in Example 1 was measured.

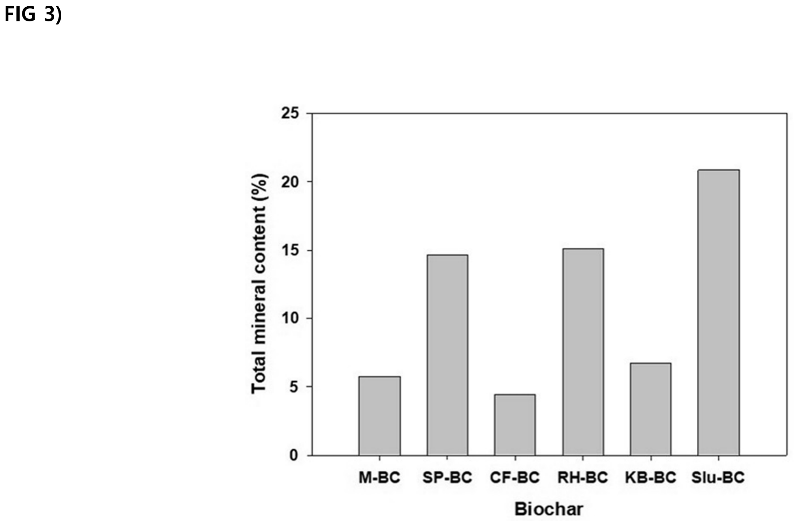

Specifically, the total mineral content was quantitatively and qualitatively analyzed using inductively coupled plasma optical emission spectrometry (ICP-OES). The analyzed minerals included nitrogen (N), phosphorus (P), potassium (K), calcium (Ca), magnesium (Mg), iron (Fe), and silicon (Si). The measured values for each mineral were summed to calculate the total mineral content per type of biochar. The results are shown in FIG. 3 and Table 1 below.

**Table 1**

| | M-BC (Leaf) | SP-BC (Microalgae) | CF-BC (Coffee) | RH-BC (Rice husk) | KB-BC (Lawn grass) | Slu-BC (Sludge) |
|---|---|---|---|---|---|---|
| Total Mineral Content (%) | 5.76 | 14.62 | 4.45 | 15.09 | 6.70 | 20.85 |

As shown in the results, the biochar prepared from microalgae, rice husk, and sludge exhibited relatively high mineral content, while the biochar derived from leaves, coffee grounds, and lawn grass had comparatively lower mineral content.

### Example 3. Measurement of Biogas Production

### 3-1. Measurement of Biogas Production Based on Biochar Addition

In the 2-phase anaerobic digestion process, where acidogenesis and methanogenesis of organic waste occurred sequentially, the amount of biogas produced was measured by comparing cases where biochar (as prepared in Example 1) was added to the acidogenic reactor versus the methanogenic reactor.

Specifically, organic wastes consisting of food waste, livestock manure, and sewage sludge were introduced into a two-phase anaerobic digester as feeds for anaerobic digestion. The feeds were first introduced into an acidogenesis reactor, where acidogenesis was carried out, and the acidogenic effluent generated in the acidogenesis reactor was subsequently introduced into a methanogenesis reactor to perform methanogenesis. The volume ratio of the acidogenic effluent to the methanogenic reaction liquid in the methanogenesis reactor was adjusted to 1 (acidogenic effluent) : 30 (methanogenic reaction liquid) (v/v). The introduction of the acidogenic effluent into the methanogenesis reactor was performed in the batch type, in accordance with the biogas collection and digestion by-product treatment of the methanogenesis reactor. Anaerobic digestion was carried out for a total of nine days, during which the temperatures of both the acidogenesis reactor and the methanogenesis reactor were maintained at 35 °C until the completion of the anaerobic digestion.

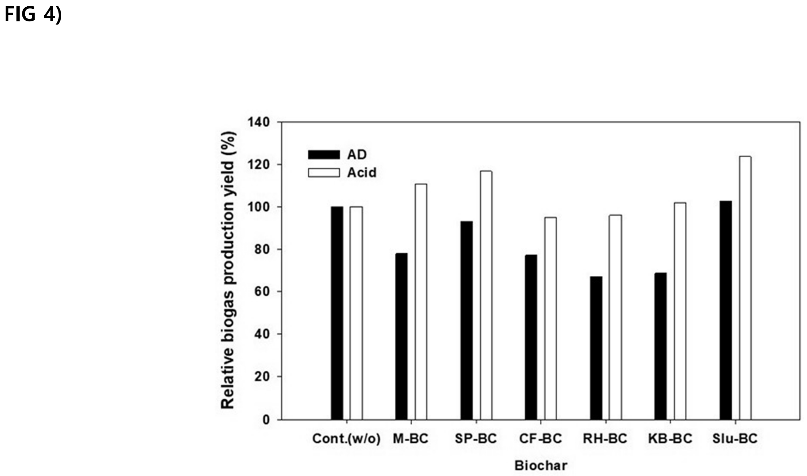

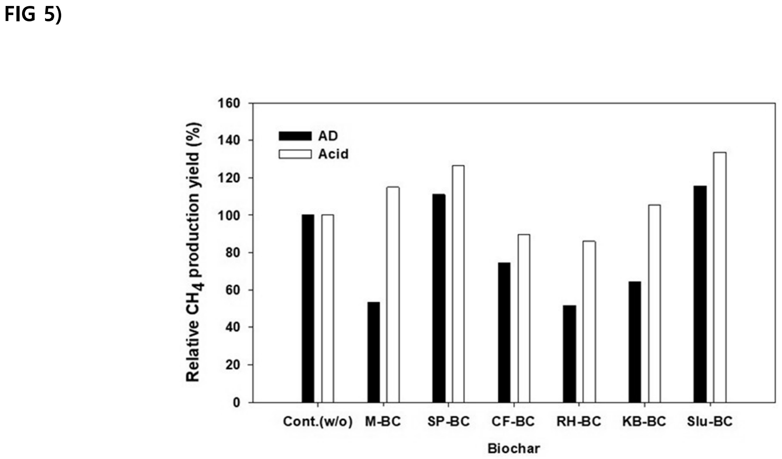

The relative biogas production was measured during the anaerobic digestion process for a control group without biochar addition (w/o) and for experimental groups in which biochar was added either to the methanogenesis reactor (AD) or to the acidogenesis reactor (Acid), and the results are shown in Fig. 4 and Table 2 below. Additionally, the relative methane production of the control group and the experimental groups was measured and presented in Fig. 5 and Table 3 below.

**Table 2**

| | Relative Biogas Production (%) According to the Addition Site of Six Types of Biochar | | | | | | |
|---|---|---|---|---|---|---|---|
| | Control | M-BC | SP-BC | CF-BC | RH-BC | KB-BC | Slu-BC |
| Methanogenesis Reactor (AD) | 100 | 77.9 | 93.0 | 77.0 | 67.0 | 68.7 | 102.6 |
| Acidogenesis Reactor (Acid) | 100 | 110.7 | 116.6 | 95.0 | 96.0 | 101.9 | 123.5 |

**Table 3**

| | Relative Methane Production (%) According to the Addition Site of Six Types of Biochar | | | | | | |
|---|---|---|---|---|---|---|---|
| | Control | M-BC | SP-BC | CF-BC | RH-BC | KB-BC | Slu-BC |
| Methanogenesis Reactor (AD) | 100 | 53.2 | 111.0 | 74.3 | 51.5 | 64.3 | 115.5 |
| Acidogenesis Reactor (Acid) | 100 | 114.9 | 126.4 | 89.7 | 86.1 | 105.5 | 133.6 |

As a result of measuring biogas and methane production, it was confirmed that, for all types of biochar, the production of biogas and methane increased when the biochar was introduced into the acidogenic reactor compared to when it was introduced into the methanogenic reactor. It was also observed that the levels of biogas and methane production varied depending on the type of biochar introduced into the anaerobic digestion reactor.

Sludge-derived biochar (Slu-BC) exhibited the highest enhancing effect on both biogas and methane production, while microalgae-derived biochar (SP-BC) and leaf-derived biochar (M-BC) also demonstrated excellent enhancement effects. On the other hand, grass-derived biochar (KB-BC), coffee ground-derived biochar (CF-BC), and rice husk-derived biochar (RH-BC) showed relatively lower enhancement in biogas or methane production compared to other types of biochar.

Given that the mineral content of the sludge- and microalgae-derived biochars was measured to be high in the mineral content analysis conducted in Example 2, it is presumed that the increase in biogas production through biochar addition during anaerobic digestion is significantly influenced by the mineral content contained in the biochar. However, in the case of the rice husk-derived biochar, despite its high mineral content, the enhancement effect on biogas production was not substantial, suggesting that in addition to mineral content, the physical properties or surface characteristics of each type of biochar may also have an influence.

Meanwhile, although the sludge-derived biochar and the microalgae-derived biochar exhibited the most significant enhancing effects on biogas and methane production, the variation in production yield according to the addition site of the biochar was more pronounced in the case of the leaf-derived biochar, rice husk-derived biochar, and grass-derived biochar. This result is considered to be attributable to the physical properties of the leaf-, rice husk-, and grass-derived biochars added to the acidogenesis reactor, which may have promoted the activity of aerobic microorganisms present therein.

Accordingly, it is presumed that the combined effects of the physical properties of the biochar promoted microbial activity in the acidogenesis reactor, thereby enhancing the production of secondary metabolites. As the acidogenic metabolites, including volatile fatty acids, were introduced into the methanogenesis reactor, they likely contributed to the growth promotion of the microorganisms involved in methanogenesis.

### 3-2. Time-Dependent Measurement of Biogas Production with Biochar Addition

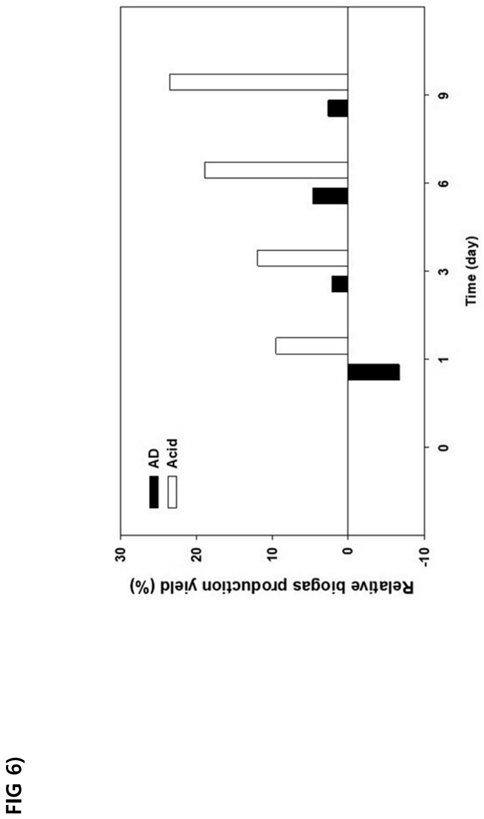

Using the sludge-derived biochar, which was confirmed to exhibit the highest enhancing effect on biogas production among the biochars prepared in Example 1, the relative biogas production in the anaerobic digestion reactor was measured over time when the sludge-derived biochar was added to either the methanogenesis reactor or the acidogenesis reactor, in comparison to a control group (w/o) in which no biochar was added. The results are shown in FIG. 6 and Table 4 below.

**Table 4**

| | Relative Biogas Production Over Time Following Addition of Sludge-Derived Biochar | | | | |
|---|---|---|---|---|---|
| | Day 0 | Day 1 | Day 3 | Day 6 | Day 9 |
| Methanogenesis Reactor | 0 | -6.7 | 2.1 | 4.6 | 2.6 |
| Acidogenesis Reactor | 0 | 9.5 | 11.9 | 18.8 | 23.5 |

As a result of the measurements, when the biochar was introduced into the methanogenesis reactor, the biogas production efficiency significantly decreased during the initial stage of anaerobic digestion, and the overall enhancement in biogas production remained minimal thereafter. In contrast, when the sludge-derived biochar was introduced into the acidogenesis reactor, biogas production continuously increased throughout the entire anaerobic digestion period, indicating that adding biochar to the acidogenic reactor provides a superior enhancement effect on biogas production compared to adding it to the methanogenic reactor.

### [EXPLANATION OF REFERENCE NUMERALS]

**1000:** Integrated Biogas Production System
**1100:** Pretreatment Unit
**1200:** Carbonization Unit
**1300:** Receiving Unit
**1400:** Acidogenesis Unit
**1500:** Methanogenesis Unit
**1600:** Collection Unit
**1700:** By-product Treatment Unit
**S1000:** Biogas Production Method
**S101:** Pretreatment Step
**S102:** Carbonization Step
**S200:** Acidogenesis Step
**S300:** Methanogenesis Step
**S400:** Collection Step
**S401:** By-product Treatment Step

## Claims

1. A two-phase anaerobic digestion system comprising: an acidogenic reactor in which acidogenesis of organic waste is performed; and
a methanogenic reactor in which methanogenesis of an acidogenic liquid generated from the acidogenic reactor is performed.

2. The system of claim 1, wherein biochar is added to the acidogenic reactor or the methanogenic reactor during the acidogenesis or methanogenesis process.

3. The system of claim 1 or 2, further comprising:
a receiving unit in which organic waste introduced as a feeds for anaerobic digestion is introduced.

4. The system of any one of claims 1 to 3, further comprising:
a pretreatment unit in which the organic waste is pretreated.

5. The system of claim 4, wherein the pretreatment unit performs one or more operations selected from the group including drying, crushing, composting, microbial decomposition, pyrolysis, screening, and sorting of the organic waste.

6. The system of any one of claims 1 to 5, further comprising:
a carbonization unit in which biochar is produced by carbonizing the organic waste.

7. The system of claim 6, wherein the carbonization unit operates at a temperature ranging from 300°C to 800°C.

8. The system of any one of claims 1 to 7, further comprising:
a collection unit for collecting biogas generated from the acidogenic reactor or the methanogenic reactor.

9. The system of claim 8, wherein the collection unit performs compression storage, purification, or upgrading of the biogas.

10. The system of any one of claims 1 to 9, further comprising:
a by-product treatment unit for treating anaerobic digestion by-products generated from the acidogenic reactor or the methanogenic reactor.

11. The system of claim 10, wherein the by-product treatment unit performs one or more operations selected from drying, composting, aging, or crushing of the anaerobic digestion by-products.

12. A two-phase anaerobic digestion method comprising:
an acidogenesis step of subjecting organic waste to acidogenesis; and
a methanogenesis step of subjecting an acidogenic liquid obtained from the acidogenesis step to methanogenesis.

13. The method of claim 12, wherein biochar is further added to the organic waste or the acidogenic liquid in the acidogenesis step or the methanogenesis step.
